(19) Europäisches Patentamt
European Patent Office
Office européen des brevets

(11) Veröffentlichungsnummer: **0 427 125 B1**

# (12) EUROPÄISCHE PATENTSCHRIFT

(45) Veröffentlichungstag der Patentschrift: **13.07.94**

(51) Int. Cl.5: **C07C 391/02**, A61K 31/165, C07D 263/10

(21) Anmeldenummer: **90121005.4**

(22) Anmeldetag: **02.11.90**

## (54) Benzylselenobenzamide von Anilinen und Benzylaminen.

(30) Priorität: **08.11.89 DE 3937169**

(43) Veröffentlichungstag der Anmeldung:
**15.05.91 Patentblatt 91/20**

(45) Bekanntmachung des Hinweises auf die Patenterteilung:
**13.07.94 Patentblatt 94/28**

(84) Benannte Vertragsstaaten:
**AT BE CH DE DK ES FR GB GR IT LI LU NL SE**

(56) Entgegenhaltungen:
**AT-B- 17 118**

(73) Patentinhaber: **A. Nattermann & Cie. GmbH**
**Nattermannallee 1**
**D-50829 Köln(DE)**

(72) Erfinder: **Evers, Michel, Dr.**
**Rue-de-Camtine 9**
**B-4000 Liége(BE)**
Erfinder: **Fischer, Hartmut, Dr.**
**Arnulfstr. 3-5**
**W-5000 Köln 41(DE)**
Erfinder: **Biedermann, Jürgen, Dr.**
**Kirschenweg 14**
**W-5024 Pulheim 3(DE)**
Erfinder: **Terlinden, Rolf, Dr.**
**Am Scheidweg 58**
**W-5000 Köln 71(DE)**
Erfinder: **Leyck, Sigurd, Dr.**
**Am Ouechenhauf 21**
**W-5024 Pulheim 2(DE)**

(74) Vertreter: **Döring, Wolfgang, Dr. Ing.**
**Mörikestrasse 18**
**D-40474 Düsseldorf (DE)**

EP 0 427 125 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Benzylselenobenzamide, Verfahren und Zwischenprodukte zur ihrer Herstellung sowie diese Verbindung enthaltende pharmazeutische Präparate.

Organische selenhaltige Verbindungen sind in der pharmazeutische Anwendung bekannt. So beschreibt beispielsweise die DE 3027075 A1 Benzisoselenazolone, die sich durch eine entzündungshemmende und antiatherosklerobische Wirksamkeit auszeichnen, wobei diese Verbindungen darüber hinaus noch eine gute Verträglichkeit besitzen.

Der vorliegenden Erfindung liegt die Aufgabe zugrunde, neue und pharmazeutisch besonders wirksame organische Selenverbindungen zur Verfügung zu stellen.

Diese Aufgabe wird durch Benzylselenobenzamide mit den kennzeichnenden Merkmalen des Patentanspruchs 1 gelöst.

Die erfindungsgemäßen Verbindungen entsprechen der allgemeinen Formel I

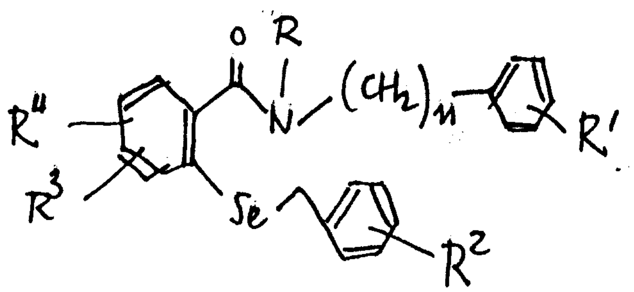

Formel I

worin

R Wasserstoff, Methyl oder Ethyl bedeutet und

$R^1$,$R^2$ gleich oder verschieden sind und unabhängig voneinander Wasserstoff, Fluor, Chlor, Brom, $C_{1-4}$-Alkyl, $C_{1-4}$ Alkoxy, Hydroxy, Cyano, Amino, Dimethylamino oder Nitro bedeuten und

$R^3$,$R^4$ gleich oder verschieden sind und unabhängig voneinander für Wasserstoff, Fluor, Chlor, Brom, $C_{1-4}$-Alkyl, $C_{1-4}$-Alkoxy, Hydroxy, Cyano, Nitro oder zusammen für Methylendioxy stehen und

n Null 1 oder 2 bedeutet.

Bevorzugt sind dabei Benzylselenobenzamide der allgemeinen Formel I, worin n Null ist und R, $R^2$, $R^3$, $R^4$ Wasserstoff bedeuten, während $R^1$ Wasserstoff, Fluor, Chlor, Brom, Methyl, Methoxy, Hydroxy, Cyano oder Nitro bedeutet.

Eine andere bevorzugte Gruppe der Benzylselenobenzamide der Formel I sind diejenigen, worin n gleich Null ist und R, $R^1$, $R^3$ und $R^4$ Wasserstoff bedeuten, während $R^2$ Wasserstoff, Fluor, Chlor, Brom, Methyl, Methoxy, Hydroxy, Cyano oder Nitro darstellt.

Ebenfalls bevorzugt sind Benzylselenobenzamide der allgemeinen Formel I, worin n gleich Null ist und R und $R^2$ Wasserstoff bedeuten, während $R^1$, $R^3$ und $R^4$ gleich oder verschieden sein können und unabhängig voneinander Wasserstoff, Fluor, Chlor, Brom, Methyl, Methoxy, Hydroxy, Cyano, Nitro, Amino, Dimethylamino oder $R^3$ und $R^4$ zusammen Methylendioxy bedeuten.

Erfindungsgemäße Verbindungen sind beispielsweise:

N-(4-Methylphenyl)-2-benzylselenobenzamid
N-(4-Methoxyphenyl)-2-benzylselenobenzamid
N-(4-Fluorophenyl)-2-benzylselenobenzamid
N-(4-N',N'-Dimethylaminophenyl)-2-benzylselenobenzamid
N-(4-Hydroxyphenyl)-2-benzylselenobenzamid
N-(4-Chlorphenyl)-2-benzylselenobenzamid
N-(4-Cyanophenyl)-2-benzylselenobenzamid
N-(4-Nitrophenyl)-2-benzylselenobenzamid
N-Ethyl-N-(4-fluorphenyl)-2-benzylselenobenzamid
R(+)-N-(-1-Phenylethyl)-2-benzylselenobenzamid
S(-)-N-(-1-Phenylethyl)-2-benzylselenobenzamid
2-Benzylselenobenzanilid
N-Phenyl-(2-(4-methylbenzylseleno))benzamid
N-Phenyl-(2-(4-methoxybenzylseleno))benzamid
N-Phenyl-(2-(4-bromobenzylseleno))benzamid

N-Phenyl-(2-(4-cyanobenzylseleno))benzamid
N-Phenyl-(2-(4-nitrobenzylseleno))benzamid
N-Phenyl-(2-(4-fluorbenzylseleno))benzamid
N-Phenyl-(2-(4-chlorbenzylseleno))benzamid
N-Phenyl-(2-(3-chlorbenzylseleno))benzamid
2-Benzylseleno-3-methoxy-benzanilid
2-Benzylseleno-3,4-methylendioxy-benzanilid
2-Benzylseleno-3-fluor-benzanilid
2-Benzylseleno-3-fluor-benzoesäure-N-ethyl-N-(4-fluorphenyl)-amid
2-Benzylseleno-3-fluor-benzoesäure-N-benzylamid
2-Benzylseleno-3-methoxy-benzoesäure-N-methyl-N-phenylamid
2-Benzylseleno-3-chlor-benzanilid

Die erfindungsgemäßen Verbindungen können aus den aktiven, isolierbaren Zwischenverbindungen der Formel II erhalten werden.

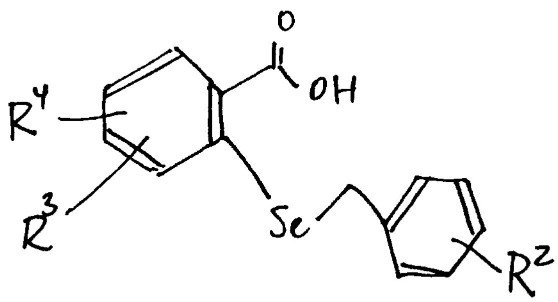

Formel II

In Formel II bedeuten

- $R^2$ Wasserstoff, Fluor, Chlor, Brom, $C_{1-4}$-Alkyl, $C_{1-4}$-Alkoxy, Hydroxy, Cyano, Amino, Dimethylamino oder Nitro, während
- $R^3$, $R^4$ gleich oder verschieden sind und unabhängig voneinander für Wasserstoff, Fluor, Chlor, Brom, $C_{1-4}$-Alkyl, $C_{1-4}$-Alkoxy, Hydroxy, Cyano, Nitro oder gemeinsam für Methylendioxy stehen.

Für die Herstellung der Verbindungen der Formel II wird eine Diselenosalicylsäure in wäßriger Lösung mit Natronlauge alkalisch gemacht, Natriumcarbonat und Natriumdithionit zugesetzt, die Reaktionsmischung mit einem Benzylbromid umgesetzt und anschließend mit Salzsäure neutralisiert, um das Endprodukt zu erhalten.

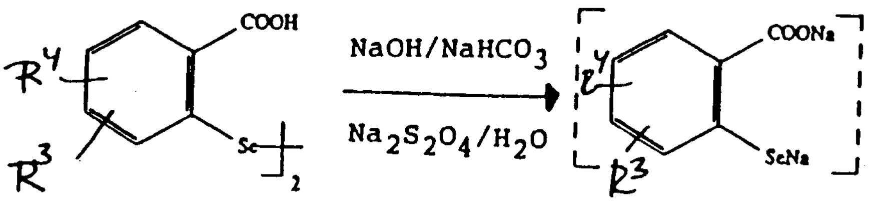


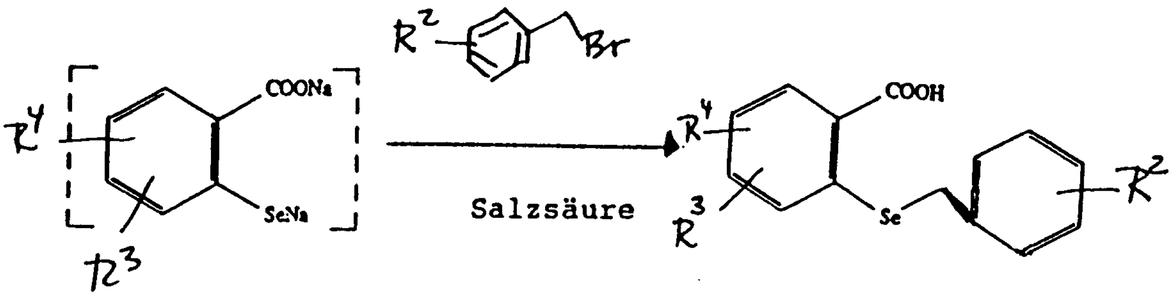


II

Die erfindungsgemäßen Verbindungen der Formel II können aber auch hergestellt werden, indem man eine Oxazolinverbindung der allgemeinen Formel V

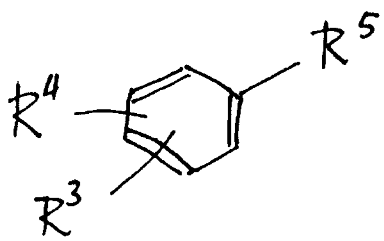

Formel V

in Tetrahydrofuran unter Zusatz von n-Butyllithium in n-Hexan mit einem Dibenzyldiselenid umsetzt, wie dies in den Beispielen 13 bis 15 näher beschrieben ist. Aus dem Reaktionsgemisch wird dann das entstehende Produkt der allgemeinen Formel III abgetrennt.

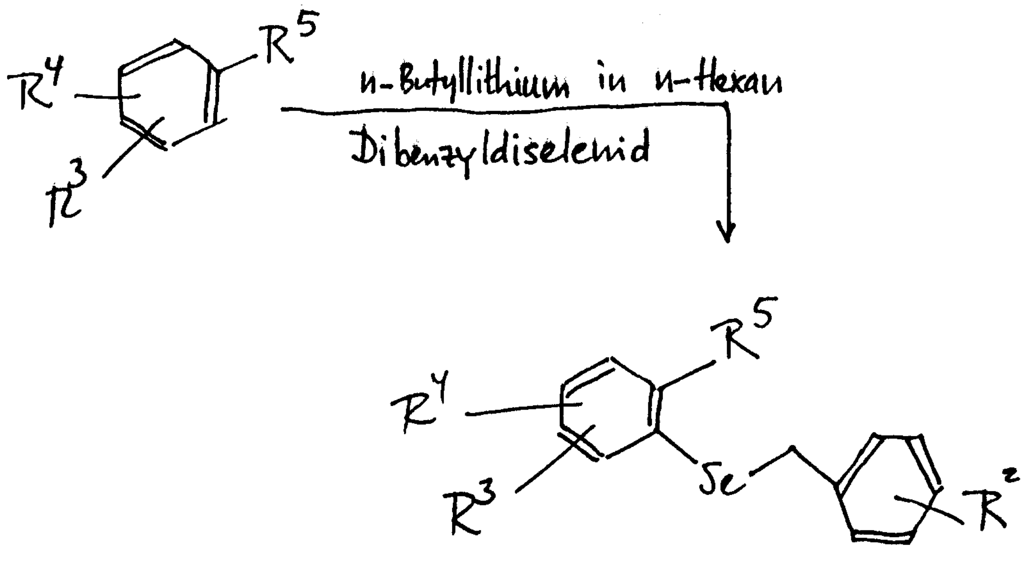


III

In den vorstehend genannten Formel III und V stehen

- $R^3$ unabhängig von $R^4$ Wasserstoff, Fluor, Chlor, Brom, $C_{1-4}$-Alkoxy oder Dimethylamin;
- $R^4$ für Wasserstoff oder zusammen mit $R^3$ für Methylendioxy; und
- $R^5$ für 1,3-Oxazolin-2-yl oder 4,4-Dimethyl-1,3-Oxazolin-2-yl.

So fällt unter die erfindungsgemäße Verbindung beispielsweise eine Verbindung, wie sie durch die nachstehend genannte Formel V a wiedergegeben wird, wobei in dieser Formel V a $R^3$ und $R^4$ die Bedeutungen besitzen, wie diese vorstehend für die Formel V genannt sind, während $R^2$ Wasserstoff, Fluor, Chlor, Brom, $C_{1-4}$-Alkyl, $C_{1-4}$-Alkoxy oder Dimethylamino ist.

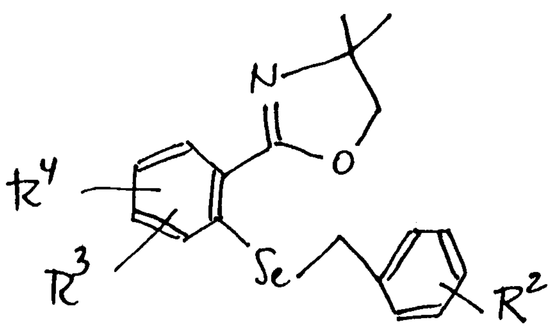

Formel V a

Geeignete Oxazolinverbindungen gemäß der Formel V sind beispielsweise:
4,4-Dimethyl-2-(3,4-methylendioxy)phenyl-1,3-oxazolin,
4,4-Dimethyl-2-(3-methoxyphenyl)-1,3-oxazolin,
4,4-Dimethyl-2-(3-fluorphenyl)-1,3-oxazolin oder
4,4-Dimethyl-2-(3-chlorphenyl)-1,3-oxazolin,
wie diese etwa von A.I. Meyers, W.B. Avila in J. Org.Chem. 46 (1981), 3881-3886 beschrieben sind.

Durch Verseifung von Verbindungen der allgemeinen Formel III erhält man schließlich die erfindungsgemäßen Verbindungen der allgemeinen Formel II, wie dies konkret in den Ausführungsbeispielen 10 bis 12 angegeben ist.

Für die Synthese der Produkte der Formel I eignen sich beispielsweise folgende Verbindungen:
2-(4-Methylbenzylseleno)benzoesäure
2-(4-Methoxybenzylseleno)benzoesäure
2-(4-Brombenzylseleno)benzoesäure
2-(4-Cyanobenzylseleno)benzoesäure
2-(4-Nitrobenzylseleno)benzoesäure
2-(4-Fluorbenzylseleno)benzoesäure
2-(3-Chlorbenzylseleno)benzoesäure
2-(4-Chlorbenzylseleno)benzoesäure

2-Benzylseleno-3-methoxy-benzoesäure
2-Benzylseleno-3,4-methylendioxybenzoesäure
2-Benzylseleno-3-fluor-benzoesäure

Die Benzylselenobenzoesäuren werden als aktive isolierbare Zwischenverbindungen zur Herstellung der Benzylselenobenzamide gemäß Formel I verwendet. Benzylselenobenzoesäuren gemäß Formel II mit n = 0 werden in einem chlorierten Kohlenwasserstoff mit Bis-(2-oxo-3-oxazolidinyl)phosphorylchlorid und einem Anilin umgesetzt und das entstandene Produkt aus dem Reaktionsgemisch abgetrennt.

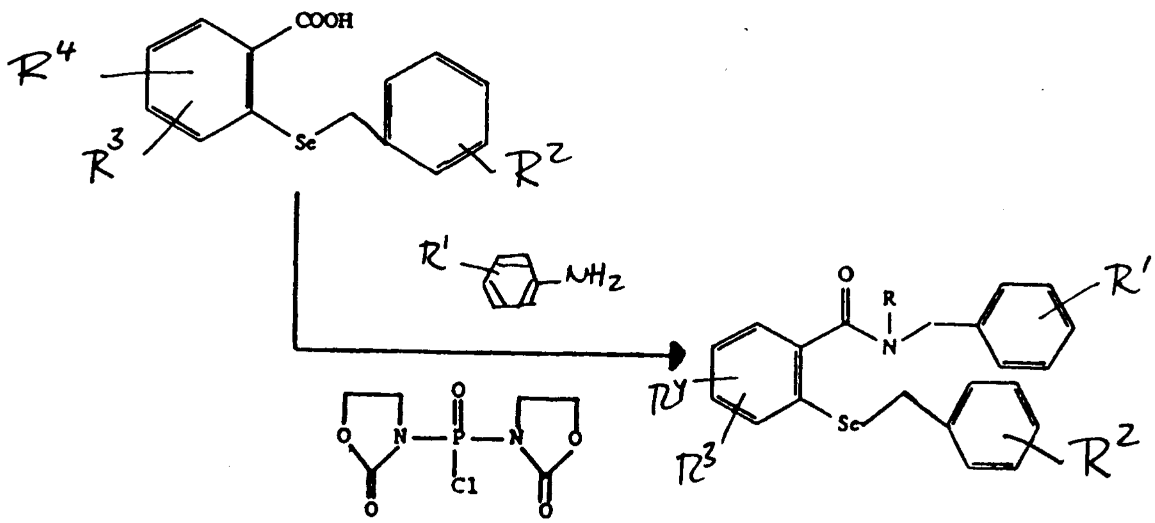

Produkte gemäß der vorstehend wiedergegebenen Formel I mit n = 1 werden wie folgt hergestellt:

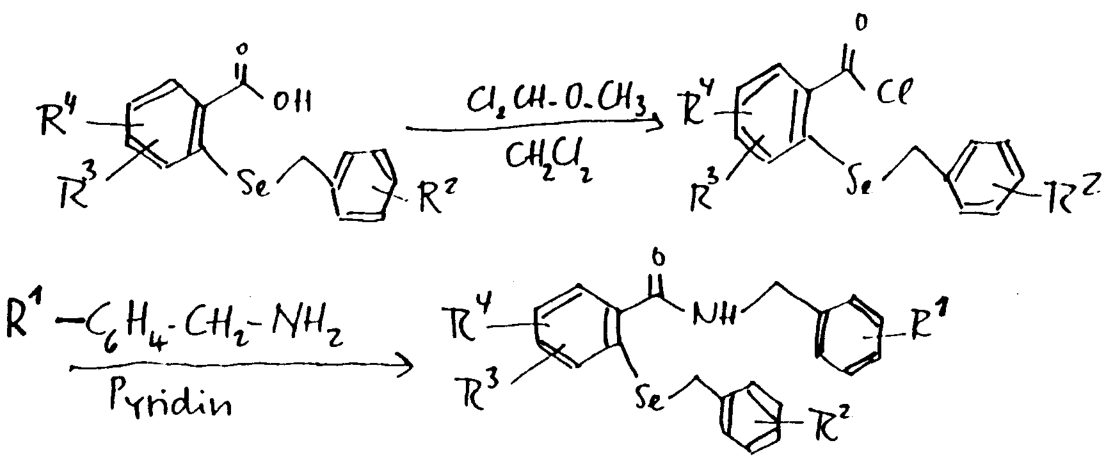

Produkte gemäß Formel I mit n = 2 werden nach folgendem Reaktionsmechanismus hergestellt:

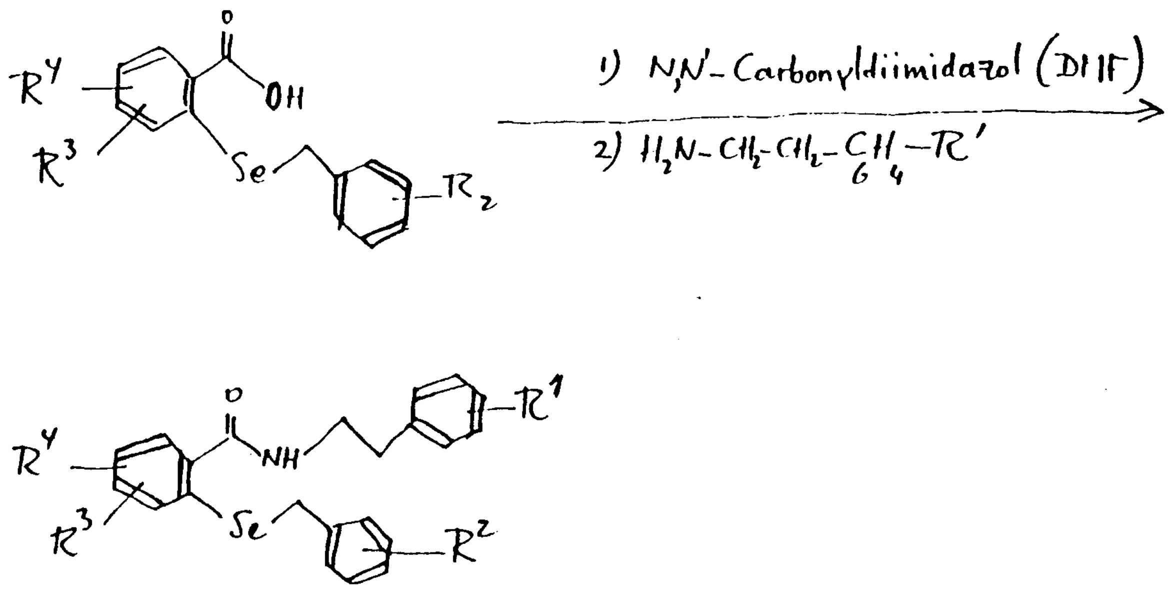


In den vorstehend wiedergegebenen Formeln haben $R^1$, $R^2$, $R^3$ und $R^4$ die Bedeutungen, wie diese vorstehend bei der Formel I angegeben sind.

Die vorliegende Erfindung betrifft ebenfalls pharmazeutische Präparate, welche Verbindungen der vorstehend genannten Formel I als Wirkstoffe enthalten. Bei den erfindungsgemäßen pharmazeutischen Präparaten handelt es sich um solche zur enteralen wie oralen oder rektalen oder parenteralen Verabreichung, welche die pharmazeutischen Wirkstoffe allein oder zusammen mit einem üblichen, pharmazeutisch anwendbaren Trägermaterial enthalten.

Vorteilhafterweise liegt die pharmazeutische Zubereitung des Wirkstoffes in Form von Einzeldosen vor, die auf die gewünschte Verabreichung abgestimmt sind, wie z.B. Tabletten, Dragees, Kapseln, Suppositorien, Granulate, Lösungen, Emulsionen oder Suspensionen. Die Dosierung der Substanzen liegt üblicherweise zwischen 10 und 1.000 mg pro Tag, vorzugsweise zwischen 30 und 300 mg pro Tag und kann in einer Dosis oder mehreren Teildosen, vorzugsweise in zwei bis drei Teildosen pro Tag, verabreicht werden. Es konnte festgestellt werden, daß ein derartiges pharmazeutisches Präparat hervorragende entzündungshemmende Eigenschaften besitzt.

Die Herstellung der erfindungsgemäßen Verbindungen wird durch die folgenden Beispiele näher erläutert.

Die angegebenen Schmelzpunkte wurden mit einem Büchi 510-Schmelzpunktbestimmungsapparat gemessen und sind mit °C angegeben und nicht korrigiert.

Beispiel 1

2-Benzylselenobenzoesäure

Zu einer Suspension von 50,0 g (0,125 Mol) Diselenosalicylsäure in 380 ml Wasser werden unter Rühren 38,0 g (0,95 Mol) Natriumhydroxid gegeben, wobei die Innentemperatur auf 45°C ansteigt und die Säure in Lösung geht. Nach Zugabe von 100 g (0,94 Mol) Natriumcarbonat und 2,58 g (0,333 Mol) Natriumdithionit steigt die Temperatur auf 55°C. Man erhitzt 2 Stunden unter Rückfluß, läßt abkühlen und tropft bei Raumtemperatur innerhalb 10 Minuten 47,6 ml (68,4 g; 0,4 Mol) Benzylbromid zu. Nach weiterer Wasserzugabe (100 ml) wird über 14 Stunden bei Raumtemperatur weitergerührt, dann werden 355 ml Salzsäure (32 %) unter Rühren zum Reaktionsgemisch langsam zugetropft, die ausgefallene weiße Festsubstanz abgesaugt und mit Wasser neutral gewaschen. Das noch feuchte Rohprodukt wird unter Zusatz von 3g Aktiv-Kohle aus 1600 ml Propanol umkristallisiert. Die Ausbeute beträgt 48,8 g (67% d. Th.). Aus der Mutterlauge werden weitere 12 g (16,5 %) 2-Benzylselenobenzoesäure gewonnen.
Ausbeute: 60,8 g (83,5% d. Th.)
Fp.: 209-210°C

Beispiel 2

2-(4-Methylbenzylseleno)benzoesäure

Zu einer Suspension von 20,1 g (0,05 Mol) Diselenosalicylsäure in 20,0 ml Wasser gibt man auf einmal unter Rühren 15 g (0,375 Mol) Natriumhydroxyd. Nach Auflösung werden 39,75 g (0,375 Mol) Natriumcarbonat und 23,9 g (0,1375 Mol) Natriumdithionit zugegeben, anschließend wird 2 Stunden unter Rückfluß erhitzt. Man läßt abkühlen und tropft innerhalb von 15 Minuten eine Lösung von 30,1 g (0,1625 Mol) 4-Methylbenzylbromid in 125 ml Ethanol zu und rührt über Nacht. Das Reaktionsgemisch wird unter Rühren mit 150 ml wässriger HCl (32%) angesäuert, der ausgefallene weiße Niederschlag abgesaugt, mit 200 ml Wasser gewaschen und getrocknet. Der Rückstand wird durch Umkristallisation aus Essigester gereinigt.
Ausbeute: 22 g (71 % d.Th.)
Fp.: 220-222 °C

Beispiel 3

2-(4-Methoxybenzylseleno)benzoesäure

Analog Beispiel 2 erhält man unter Verwendung von 25,4 g (0,1625 Mol) 4-Methoxybenzylchlorid als Reaktionsprodukt 2-(4-Methoxybenzylseleno)benzoesäure.
Ausbeute: 27,0 g (85 % d.Th.)
Fp.: 225-228 °C

Beispiel 4:

2-(4-Brombenzylseleno)benzoesäure

Analog Beispiel 2 erhält man unter Verwendung von 40,6 g (0,1625 Mol) 4-Brombenzylbromid als Reaktionsprodukt 2-(4-Brombenzylseleno)benzoesäure.
Ausbeute: 31,4 g (84,7 % d.Th.)
Fp.: 255-258 °C

Beispiel 5

2-(4-Cyanobenzylseleno)benzoesäure

Analog Beispiel 2 aus:
10 g (0,052 Mol) 4-(Brommethyl)benzonitril in 100 ml Ethanol
6,3g (0,016 Mol) Diselenosalicylsäure,
4,8 g (0,12 Mol) Natriumhydroxyd,
12,7 g ( 0,12 Mol) Natriumcarbonat,
7,7 g (0,044 Mol) Natriumdithionit und
100 ml destilliertem Wasser
Ausbeute: 7,4 g (73 % d.Th.)
Fp.: 252-254 °C

Beispiel 6

2-(4-Nitrobenzylseleno)benzoesäure

Analog Beispiel 2 aus:
22,3 g (0,13 Mol) 4-Nitrobenzylchlorid in 250 ml Ethanol
16 g (0,04 Mol) Diselenosalicylsäure,
12 g (0,3 Mol) Natriumhydroxyd,
32 g (0,3 Mol) Natriumcarbonat,
18,4 g (0,11 Mol) Natriumdithionit und
300 ml destilliertem Wasser
Ausbeute: 13,7 g (72 % d.Th.)

Fp.: 232-235 ° C

Beispiel 7

2-(4-Fluorbenzylseleno)benzoesäure

Analog Beispiel 2 aus:
23,5 g (19,5 ml; 0,16 Mol) 4-Fluorbenzylchlorid
20 g ( 0,05 Mol) Diselenosalicylsäure,
15 g (0,38 Mol) Natriumhydroxyd,
40 g (0,39 Mol) Natriumcarbonat,
24 g (0,14 Mol) Natriumdithionit und
400 ml destilliertem Wasser
Ausbeute: 22 g (71 % d.Th.)
Fp.: 220-222 ° C

Beispiel 8

2-(3-Chlorbenzylseleno)benzoesäure

Analog Beispiel 2 aus:
12 g (0,07 Mol) 3-Chlorbenzylchlorid (unverdünnt)
8 g (0,02 Mol) Diselenosalicylsäure,
6 g (0,15 Mol) Natriumhydroxyd,
16 g (0,15 Mol) Natriumcarbonat,
9,2 g (0,05 Mol) Natriumdithionit und
150 ml destilliertem Wasser
Ausbeute: 7,9 g (61 % d.Th.)
Fp.: 209-211 ° C

Beispiel 9

2-(4-Chlorbenzylseleno)benzoesäure

Analog Beispiel 2 aus:
20,9 g (0,13 Mol) 4-Chlorbenzylchlorid in 100 ml Ethanol
16 g (0,04 Mol) Diselenosalicylsäure,
12 g (0,3 Mol) Natriumhydroxyd,
32 g (0,3 Mol) Natriumcarbonat,
18,4 g (0,11 Mol) Natriumdithionit und
150 ml destilliertem Wasser
Ausbeute: 21 g (80,6 % d.Th.)
Fp.: 234-237 ° C

Beispiel 10

2-Benzylseleno-3-methoxybenzoesäure

Eine Suspension von
13,5 g (0,0362 Mol) 4,4-Dimethyl-2-(2-benzylseleno--3-methoxyphenyl)-1,3-oxazolin in
21,5 ml (0,181 Mol) Benzylbromid wird bei Raumtemperatur 24 Stunden gerührt und die Lösung eingeengt. Der Rückstand wird in einem Gemisch aus 1,8 l Natronlauge (20%) und 1,8 l Methanol gelöst und 12 h am Rückfluß erwärmt, der Kolbeninhalt mit 300 ml Diethylether extrahiert und die so erhaltene organische Phase verworfen. Die wäßrige Phases wird mit konz. Salzsäure auf pH 1 gestellt, der ausgefallene Feststoff abgesaugt mit 200 ml Wasser gewaschen und getrocknet.
Ausbeute: 6,9 g (59,6% d. Th.)
Fp.: 151-153 ° C

Beispiel 11

2-Benzylseleno-3,4-methylendioxybenzoesäure

Analog Beispiel 10 aus:
2,45 g (0,0063 Mol) 4,4-Dimethyl-2-(2-benzylseleno--3,4-methylendioxyphenyl)-1,3-oxazolin in
5,4 g (0,032 Mol) Benzylbromid
Ausbeute: 1,6 g (75,8% d.Th.)
F.p.: 196-197 °C

Beispiel 12

2-Benzylseleno-3-fluorbenzoesäure

Analog Beispiel 10 aus:
43,5 g (0,12 Mol) 4,4-Dimethyl-2-[(2-benzylseleno-3-fluor)-phenyl]-1,3-oxazolin in
102,6 g (0,6 Mol) Benzylbromid
Der etherische Rückstand wird nach der Extraktion jedoch nicht vervorfen, sondern nochmals in Benzylbromid gelöst und wie in Beispiel 10 aufgearbeitet. Nach Einstellung der wäßrigen Phase auf pH 1 wird zuerst mit Wasser und nach Trocknung dann mit n-Hexan nachgewaschen.
Gesamtausbeute: 29,9 g ( 80,6 % d.Th.)
Fp.: 137-138 °C

Beispiel 13

4,4-Dimethyl-2-[(2-benzylseleno-3-fluor)-phenyl]-1,3-oxazolin

Zu einer auf -45 °C abgekühlten Lösung von 30,0 g 4,4-Dimethyl-2-(3-fluorphenyl)1,3-oxazolin in 400 ml getrocknetem Tetrahydrofuran werden 100 ml einer 1,6 M Lösung n-Butyllithium in Hexan so zugetropft, daß eine Temperatur von -40 °C nicht überstiegen wird (25 Minuten). Nach zweistündigem Rühren bei -45 °C wird das Reaktionsgemisch auf 0 °C erwärmt und anschließend eine Lösung von 54,7 g (0,16 Mol) Dibenzyldiselenid in 250 ml getrocknetem Tetrahydrofuran innerhalb von 20 Minuten zugetropft. Das Reaktionsgemisch wird 18 Stunden bei Raumtemperatur gerührt. Der Kolbeninhalt wird in 300 g Eis und 300 ml Wasser aufgenommen und 2mal mit je 300 ml Diethylether extrahiert. Die organische Phase wird nacheinander mit 200 ml Wasser, 200 ml 10 %iger Natriumhydrogencarbonat-Lösung und 200 ml Wasser ausgeschüttelt, über Natriumsulfat getrocknet und im Vakuum eingedampft. Das Rohprodukt (58,3 g) wird durch Säulenchromatographie(Kieselgel/Dichlormethan) gereinigt.
Ausbeute: 43,5 g (75 % d.Th.)
Fp.: 95 °

Beispiel 14

4,4-Dimethyl-2-[(2-benzylseleno-3-methoxy)-phenyl]-1,3-oxazolin

Analog Beispiel 13 aus:
50 g (0,244 Mol) 4,4-Dimethyl-2-(3-methoxyphenyl)-1,3-oxazolin in 500 ml getrocknetem Tetrahydrofuran
167,5 ml (0,268 Mol) 1,6 M Lösung von n-Butyllithium in Hexan
91 g (0,268 Mol) Dibenzyldiselenid
Ausbeute: 28 g (30,7% d.Th.)
DC ($SiO_2$): $R_f$ 0,23 ($CH_2Cl_2$)

Beispiel 15

4,4-Dimethyl-2-[(2-benzylseleno-3,4-methylendioxy)-phenyl]-1,3-oxazolin

Zu einer auf 0 °C abgekühlten Lösung von 4,4 g (0,2 Mol) 4,4-Dimethyl-2-(3,4-methylendioxyphenyl)-1,3-oxazolin in 100 ml Tetrahydrofuran (getrocknet) werden 25 ml einer 1,6 M n-Butyllithium-Lösung in Hexan innerhalb einer Stunde zugetropft. Nach 30 Stunden bei 0 °C wird eine Lösung von 6,2 g (0,02 Mol)

Dibenzyldiselenid in 100 ml Tetrahydrofuran (getrocknet) zugetropft. Man rührt 2 Stunden bei 0 °C und versetzt mit 20 ml Wasser, dann wird der Kolbeninhalt im Vakuum eingedampft und mit 100 ml Dichlormethan und 100 ml Wasser aufgenommen. Die organische Phase wird abgetrennt und unter Vakuum eingedampft. Das Rohprodukt wird durch Säulenchromatographie(Kieselgel/Dichlormethan) gereinigt.
Ausbeute: 2,45 g (31,6% d.Th.)
Fp.: 107-109 °C

Beispiel 16

N-(4-Methylphenyl)-2-benzylselenobenzamid

Eine Suspension von 4 g (0,013 Mol) 2-Benzylselenobenzoesäure in 80 ml Dichlormethan wird nach Kühlen auf 0-5 °C auf einmal und unter Rühren mit 2 g (0,015 Mol) (Chlormethylen)-dimethyliminiumchlorid (Vilsmeier Reagenz) versetzt. Nach 1 Stunde wird in der Kälte eine Lösung von 1,47 g (0,0137 Mol) 4-Methylanilin und 3 g (0,03 Mol) Triethylamin zugetropft und 2 Stunden bei Raumtemperatur gerührt. Die Lösung wird nacheinander mit
100 ml 1 N wäßriger Salzsäure
100 ml 1 N Natronlauge und
100 ml destilliertem Wasser
gewaschen, mit $Na_2SO_4$ getrocknet und im Vakuum eingedampft. Der Rückstand wird aus Isopropanol umkristallisiert.
Ausbeute: 2,56g (49% d.Th.)
Fp.: 165-166 °C

Beispiel 17

N-(4-Methoxyphenyl)-2-benzylselenobenzamid

Analog Beispiel 16 erhält man bei Verwendung von
1,69 g (0,0137 Mol) 4-Methoxyanilin anstelle von 4-Methylanilin das Produkt N-(4-Methoxyphenyl)-2-benzylselenobenzamid
Ausbeute: 3,73 g (69,1% d.Th.)
Fp.: 182-183 °C

Beispiel 18

N- (4-Fluorphenyl)-2-benzylselenobenzamid

Analog Beispiel 16 erhält man unter Verwendung von 1,52 g 4-Fluoranilin (0,0137 Mol) als Produkt N-(4-Fluorphenyl)-2-benzylselenobenzamid.
Ausbeute: 2,50 g (47,4 % d.Th)
FP.: 129-131 °C

Beispiel 19

N-[4-(N',N'-Dimethylamino)phenyl]-2-benzylselenobenzamid

Analog Beispiel 16 erhält man unter Verwendung von 1,87 g (0,0137 Mol) 4-N,N-Dimethylaminoanilin eine Dichlormethan-Lösung, die dreimal mit je 100 ml destilliertem Wasser gewaschen, mit Natriumsulfat getrocknet und im Vakuum eingedampft wird.
Das Rohprodukt wird durch Umkristallisation aus
2-Propanol gereinigt.
Ausbeute: 1,77g (31,7 % d.Th.)
Fp.: 163-165 °C

Beispiel 20

N-(4-Hydroxyphenyl)-2-benzylselenobenzamid

Analog Beispiel 16 erhält man unter Verwendung von 1,5 g (0,0137 Mol) 4-Aminophenol eine Dichlormethanlösung, die mit 200 ml 1 N Salzsäure und zweimal 200 ml destilliertem Wasser gewaschen, mit Natriumsulfat getrocknet und im Vakuum eingedampft wird. Der Rückstand wird durch Säulenchromatographie (Kieselgel/Dichlormethan) gereinigt.
Ausbeute: 1,31 g (25% d.Th.)
Fp.: 139-140 °C

Beispiel 21

2-Benzylselenobenzanilid

Eine Suspension von
6 g (0,0206 mMol) 2-Benzylselenobenzoesäure in 60 ml Dichlormethan wird bei -10 °C mit 2,64 g (0,0206 Mol) (Chlormethylen)-dimethyliminiumchlorid (Vilsmeier-Reagenz) versetzt. Nach 30-minütigem Rühren bei 0 °C wird zu der klaren Reaktionsmischung eine Lösung von
1,9 g (0,04 Mol) Anilin und
4,21 g (0,0416 Mol) Triethylamin in 60 ml Dichlormethan innerhalb von 15 Minuten zugetropft. Nach 48-stündigem Rühren bei Raumtemperatur wird die klare, gelbe Lösung mit 1 N HCl (100 ml) und anschließend mit Wasser bis zur neutralen Reaktion ausgeschüttelt, die organische Phase über $Na_2SO_4$ getrocknet und nach Filtration im Vakuum bis zur Trockene eingeengt. Das feste Rohprodukt wird aus
2-Propanol (220 ml) umkristallisiert.
Ausbeute: 5,5 g (72,9 % d.Th.)
Fp.: 132-133 °C

Beispiel 22

N-(4-Chlorphenyl)-2-benzylselenobenzamid

Zu einer auf 0-5 °C abgekühlten Lösung von
6 g (0,0206 Mol) 2-Benzylselenobenzoesäure und
2,1 g (0,0206 Mol) Triethylamin in 100 ml Dichlormethan gibt man in fester Form und auf einmal
5,24 g (0,0206 Mol) Bis-[2-oxo-3-oxazolidinyl]phosphorylchlorid. Nach 30 Minuten Rühren werden
2,63 g (0,0206 Mol) 4-Chloranilin bei 0 °C zugegeben und nach 15 Minuten eine Lösung von 2,1 g (0,0206 Mol) Triethylamin in 30 ml Dichlormethan (innerhalb 2 1/2 Stunden). Das Reaktionsgemisch wird anschließend sofort mit 100 ml 1 N wässriger Salzsäure, 100 ml 1 N Natronlauge und 100 ml destilliertem Wasser ausgeschüttelt. Die organische Phase wird mit Natriumsulfat getrocknet und im Vakuum abgedampft. Der Rückstand wird durch Umkristallisation aus 80 ml
2-Propanol gereinigt.
Ausbeute: 6,60 g (79,9 % d.Th.)
Fp.: 160-161 °C

Beispiel 23

N-(4-Cyanophenyl)-2-benzylselenobenzamid

Analog Beispiel 22 erhält man unter Verwendung von 2,43 g (0,0206 Mol) 4-Aminobenzonitril (statt 4-Chloranilin) ein Rohprodukt, das einmal aus 80 ml 2-Propanol umkristallisiert wird. Die so erhaltenen Kristalle werden verworfen. Die Mutterlauge wird mit 80 ml n-Hexan versetzt. Das abfiltrierte ausgefallene Material wird durch Säulenchromatographie (Kieselgel/Dichlormethan) gereinigt.
Ausbeute: 1,54 g (19,1 % d.Th.)
FP.: 153-154 °C

Beispiel 24

N-(4-Nitrophenyl)-2-benzylselenobenzamid

Analog zu Beispiel 23 erhält man unter Verwendung von
2,84 g (0,0206 Mol) 4-Nitroanilin als Produkt N-(4-Nitrophenyl)-2-benzylselenobenzamid.
Ausbeute: 1,33 g (15,7 % d.Th.)
Fp.: 195-196 °C

Beispiel 25

N-Ethyl-N-(4-fluorphenyl)-2-benzylselenobenzamid

Analog Beispiel 22 aus:
3,0 g (0,0103 Mol) 2-Benzylselenobenzoesäure
1,44 ml Triethylamin in 20 ml $CH_2Cl_2$
2,26 g (0,0103 Mol) Bis-(2-oxo-3-oxazolidinyl)phosphorylchlorid
1,43 g (0,0103 Mol) N-Ethyl-4-fluoranilin
1,44 ml Triethylamin in 10 ml Dichlormethan
Nach der letzten Zugabe läßt man jedoch 18 Stunden bei Raumtemperatur stehen, und schüttelt dann zweimal mit je 100 ml Wasser aus. Die Dichlormethanphase wird wie bei Beispiel 22 weiterbehandelt und das Rohprodukt anschließend durch Säulen-chromatographie (Kieselgel/Dichlormethan) gereinigt.
Ausbeute: 1,25 g (29,5% d.Th.)
Fp.: 121-122 °C

Beispiel 26

R(+)-N-(1-Phenylethyl)-2-benzylselenobenzamid

Zu einer Suspension von 7,28 g (0,025 Mol) 2-Benzylselenobenzoesäure in 100 ml Dimethylformanid werden 4,45 g (0,0275 Mol) N,N'-Carbonyldiimidazol gegeben und die nach 5 Minuten klare Lösung bei Raumtemperatur bis zur Beendigung der $CO_2$-Entwicklung gerührt. Nach Zugabe von 3,03 g (0,025 Mol) R-(+)-1-Phenylethylamin wird 1 h unter Rückfluß erhitzt und anschließend 5 h bei Raumtemperatur weitergerührt.

Es wird im Vakuum eingeengt, der Rückstand in 100 ml Dichlormethan gelöst und diese Lösung nacheinander mit
100 ml 1N Salzsäure,
100 ml 1N Natronlauge und
100 ml Wasser
ausgeschüttelt. Die organische Phase wird über $Na_2SO_4$ getrocknet, filtriert und im Vakuum eingeengt.
Der im Dünnschicht-Chromatogramm ($CH_2Cl_2$ : MeOH = 9 : 1) uneinheitiiche Rückstand (10 g festes Produkt) wird mittels Flash-Chromatographie gereinigt, die im DC einheitlichen Eluate eingeengt und aus Toluol umkristallisiert.
Für die Flash-Chromatographie wird als Elutionsmittel Dichlormethan und als stationäre Phase Flash-Kieselgel verwendet.
Ausbeute: 5,7 g (57,8 %d.Th.)
Fp.: 105 - 107 °C
$[\alpha]_D^{20}$ : +67,5 ° (c=1, EtOH)

Beispiel 27

S(-)-N-(1-Phenylethyl)-2-benzylselenobenzamid

Analog Beispiel 26 erhält man den optischen Antipoden durch die Verwendung von 3,03 g (0,025 Mol) S(-)-1-Phenylethylamin.
Ausbeute: 4,9 g (49,7% d.Th.)
Fp.: 105 - 107 °C
$[\alpha]_D^{20}$ : -68,6 ° (c=1, EtOH)

Beispiel 28

N-Phenyl-[2-(4-methylbenzylseleno)]benzamid

Zu einer auf 0 °C abgekühlten Lösung von 1,58 g (0,00517 Mol) 2-(4-Methylbenzylseleno)benzoesäure (Beispiel 2) und 0,58 g (0,00573 Mol) Triethylamin in 10 ml Dichlormethan gibt man fest und auf einmal 1,32 g (0,00517 Mol) Bis[2-oxo-3-oxazolidinyl]phosphorylchlorid. Nach 30 Minuten Rühren werden nacheinander bei 0 °C eine Lösung von 0,48 g Anilin (0,47 ml; 0,00517 Mol) in 10 ml Dichlormethan und 0.74 g Triethylamin (1,02 ml; 0,00573 Mol) in 10 ml Dichlormethan zugetropft. Das Reaktionsgemisch wird bei Raumtemperatur 20 Stunden gerührt und mit 20 ml destilliertem Wasser versetzt. Die organische Phase wird abgetrennt und nacheinander mit
20 ml Wasser, zweimal mit 25 ml 10 %iger Salzsäure und zweimal mit 25 ml 10 %iger Natriumhydroxyd-Lösung gewaschen und getrocknet. Anschließend wird eingeengt und der Rückstand durch Säulenchromatographie (Kieselgel/Dichlormethan) gereinigt.
Ausbeute: 1,9 g (96 % d.Th.)
Fp.: 140 °C

Beispiel 29

N-Phenyl-[2-(4-methoxybenzylseleno)]benzamid

Analog Beispiel 28 erhält man unter Verwendung von 1,66 g (0,00517 Mol) 2-(4-Methoxybenzylseleno)-benzoesäure (Beispiel 3) als Produkt N-Phenyl-[2-(4-methoxybenzylseleno)]benzamid.
Ausbeute: 1,8 g (88 % d.Th.)
Fp. 132-133 °C

Beispiel 30

N-Phenyl-[2-(4-brombenzylseleno)]benzamid

Analog Beispiel 28 erhält man unter Verwendung von 1,92 g (0,00517 Mol) 2-(4-Brombenzylseleno)-benzoesäure (Beispiel 4) als Produkt N-Phenyl-[2-(4-brombenzylseleno)]benzamid.
Ausbeute: 2 g (87 % d.Th.)
Fp. 155 °C

Beispiel 31

N-Phenyl-[2-(4-cyanobenzylseleno)]benzamid

Analog Beispiel 28 erhält man unter Verwendung von 1,63 g (0,00517 Mol) 2-(4-Cyanobenzylseleno)-benzoesäure (Beispiel 5) als Produkt N-Phenyl-[2-(4-cyanobenzylseleno)]benzamid.
Ausbeute: 1,78g (88 % d.Th.)
Fp.: 148-149 °C

Beispiel 32

N-Phenyl-[2-(4-nitrobenzylseleno)]benzamid

Analog Beispiel 28 erhält man unter Verwendung von 1,74 g (0,00517 Mol) 2-(Nitrobenzylseleno)-benzoesäure (aus Beispiel 6) als Produkt N-Phenyl-[2-(4-nitrobenzylseleno)]benzamid.
Ausbeute: 1,8 g (84,7 % d.Th.)
Fp.: 107-108 °C

Beispiel 33

N-Phenyl-[2-(4-fluorbenzylseleno)]benzamid

Analog Beispiel 28 erhält man unter Verwendung von 1,6 g (0,00517 Mol) 2-(4-Fluorbenzylseleno)-benzoesäure (Beispiel 7) als Produkt N-Phenyl-[2-(3-fluorbenzylseleno)]benzamid.
Ausbeute: 1,44 g (72,5 % d.Th.)
Fp.: 131-132 °C

Beispiel 34

N-Phenyl-[2-(4-chlorbenzylseleno)]benzamid

Analog Beispiel 28 erhält man unter Verwendung von 1,68 g (0,00517 Mol) 2-(4-Chlorbenzylseleno)-benzoesäure (Beispiel 9) als Produkt N-Phenyl-[2-(4-chlorbenzylseleno)]benzamid.
Ausbeute: 1,68 g (81,1 % d.Th.)
Fp.: 151-152 °C

Beispiel 35

2-Benzylseleno-3-fluorbenzoesäure-N-ethyl-N-(4-fluorphenyl)-amid

Zu einer auf 0 °C abgekühlten Lösung von 0,47 g (0,0036 Mol) N-Ethyl-4-fluoranilin in 10 ml Pyridin wird unter Rühren eine Lösung von 1,1 g (0,0036 Mol) 2-Benzylseleno-3-fluorbenzoylchlorid zugetropft. Diese wurde zuvor aus 2-Benzylseleno-3-fluorbenzoesäure (Beispiel 12) und $\alpha$,$\alpha$-Dichlormethyl-methylether in Dichlormethan bei Raumtemperatur hergestellt.
Nach 4 Stunden Rühren bei Raumtemperatur wird der Kolbeninhalt auf 200 g Eis und 100 ml Wasser gegossen, 3 mal mit je 100 ml Dichlormethan extrahiert und die vereinigten Dichlormethan-Phasen nach Trocknen über $Na_2SO_4$ und Filtration im Vakuum eingeengt. Der feste Rückstand (1,5 g) wird in 100 ml Hexan ausgerührt, abgesaugt und getrocknet, man erhält 1,05 g Festsubstanz. Da im DC noch nicht einheitlich, wird die Festsubstanz nochmals in 150 ml Dichlormethan gelöst, 2 mal mit je 50 ml Soda-Lösung (10%ig) ausgeschüttelt, die organische Phase nach Trocknung über $Na_2SO_4$ eingeengt und der neuerliche, feste Rückstand aus 50 ml Hexan/Dichlormethan (1:1) umkristallisiert.
Ausbeute: 0,85 g (58,8%)
Fp.: 88-89 °C

Beispiel 36

2-Benzylseleno-3-fluorbenzoesäure-N-benzylamid

Analog Beispiel 35 erhält man die Titelverbindung bei der Umsetzung von
1,27 g (0,00388 Mol) 2-Benzylseleno-3-fluorbenzoylchlorid und
0,42 g (0,00388 Mol) Benzylamin in 50 ml Pyridin.
Ausbeute: 0,82 g (53,1% d.Th.)
Fp. 128-129 °C

Beispiel 37

2-Benzylseleno-3-methoxybenzoesäure-N-methyl-N-phenylamid

Analog Beispiel 28 erhält man unter Verwendung von 2 g (0,0062 Mol) 2-Benzylseleno-3-methoxybenzoesäure (Beispiel 10) als Produkt 2-Benzylseleno-3-methoxybenzoesäure-N-methyl-N-phenylamid.
Ausbeute: 1,25 g (50% d.Th.)
Fp.: 111 °C

Beispiel 38

2-Benzylseleno-3-fluorbenzanilid

Analog Beispiel 28 erhält man unter Verwendung von 1,6 g (0,0052 Mol) 2-Benzylseleno-3-fluorbenzoesäure (Beispiel 12) als Produkt 2-Benzylseleno-3-fluoranilid
Ausbeute: 1,5 g (78% d.Th.)
Fp.: 138 °C

Beispiel 39

2-Benzylseleno-3-methoxy-benzanilid

Zu einer Lösung von 1,37 g (0,0041 Mol) 2 Benzylseleno-3-methoxybenzoylchlorid (hergestellt aus 2-Benzylseleno-3-methoxybenzoesäure und , -Dichlormethylether in Dichlormethan bei Raumtemperatur) in 10 ml Pyridin, wird bei 0 °C eine Lösung von 0,38 g (0,0041 Mol - 0,37 ml) Anilin zugetropft. Nach 18 Stunden Rühren bei Raumtemperatur wird der Kolbeninhalt auf 150 g Eis und 50 ml 25%ige HCl gegossen und extrahiert. Die organische Phase wird mit 50 ml 10%ige $NaHCO_3$ und 2 mal mit je 100 ml Dichlormethan und 200 ml Wasser gewaschen, über Natriumsulfat getrocknet und im Vakuum eingedampft.

Der Rückstand wird durch Säulenchromatographie (Kieselgel/Dichlomethan) gereinigt. Man erhält ein Öl
IR (KBr) 1667 (m), 755 $cm^{-1}$(S).
Ausbeute: 1,1 g (68,5% d.Th.)

Beispiel 40

2-Benzylseleno-3,4-methylendioxybenzanilid

Zu einer bei 0 °C abgekühlten Lösung von 1,6 g (0,0048 Mol) 2-Benzylseleno-3,4-methylendioxybenzoesäure und 0,48 g Triethylamin in 50 ml Dichlormethan, gibt man in fester Form und auf einmal 1,22 g (0,0048 Mol) Bis-[2-oxo-3-oxazolidinyl]phosphorylchlorid. Nach 30 Min. Rühren wird 0,44 g (0,44 ml) Anilin in 5 ml Dichlormethan bei 0 °C zugetropft und nach 1 Stunde eine Lösung von 0,48 g (0,66 ml) Triethylamin in 10 ml Dichlormethan. Nach 90 Minuten bei Raumtemperatur wird das Reaktionsgemisch nacheinander mit 50 ml 1 N HCl und 50 ml Wasser ausgeschüttelt. Die organische Phase wird über Natriumsulfat getrocknet und im Vakuum eingedampft. Das rohe Produkt wird durch Umkristallisation aus 2-Propanol gereinigt.
Ausbeute: 0,7 g (35,9% d.Th.)
Fp.: 141-142 °C

Beispiel 41

N-Phenyl-[2-(3-chlorbenzylseleno)]benzamid

Analog Beispiel 17 erhält man unter Verwendung von 1,68 g (0,00517 Mol) 2-(3-Chlorbenzylseleno)-benzoesäure (Beispiel 8) als Produkt N-Phenyl-[2-(3-chlorbenzylseleno)]benzamid.
Ausbeute: 1,6 g (77,2 % d.Th.)
Fp.: 131-132 °C

## Patentansprüche

1. Benzylselenobenzamide der allgemeinen Formel I

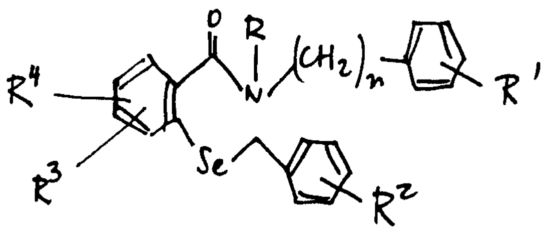

(Formel I)

worin

| | |
|---|---|
| R | Wasserstoff, Methyl oder Ethyl bedeutet und |
| $R^1$, $R^2$ | gleich oder verschieden sind und unabhängig voneinander Wasserstoff, Fluor, Chlor, Brom, $C_{1-4}$-Alkyl, $C_{1-4}$-Alkoxy, Hydroxy, Cyano, Amino, Dimethylamino oder Nitro bedeuten und |
| $R^3$, $R^4$ | gleich oder verschieden sind und unabhängig voneinander für Wasserstoff, Fluor, Chlor, Brom, $C_{1-4}$-Alkyl, $C_{1-4}$-Alkoxy, Hydroxy, Cyano, Nitro oder zusammen für Methylendioxy stehen und |
| n | Null, 1 oder 2 bedeutet. |

2. Benzylselenobenzamide gemäß Formel I, Anspruch 1, worin

| | |
|---|---|
| n | gleich Null ist und |
| R, $R^2$, $R^3$ und $R^4$ | Wasserstoff bedeuten, während |
| $R^1$ | Wasserstoff, Fluor, Chlor, Brom, Methyl, Methoxy, Hydroxy, Cyano oder Nitro bedeutet. |

3. Benzylselenobenzamide der allgemeinen Formel I, Anspruch 1, worin

| | |
|---|---|
| n | gleich Null ist und |
| R, $R^1$, $R^3$ und $R^4$ | Wasserstoff bedeuten, während |
| $R^2$ | Wasserstoff, Fluor, Chlor, Brom, Methyl, Methoxy, Hydroxy, Cyano oder Nitro bedeutet. |

4. Benzylselenobenzamide der allgemeinen Formel I, Anspruch 1, worin

| | |
|---|---|
| n | gleich Null ist und |
| R, $R^2$ | Wasserstoff bedeuten, während |
| $R^1$, $R^3$ und $R^4$ | gleich oder verschieden sein können und unabhängig voneinander Wasserstoff, Fluor, Chlor, Brom, Methyl, Methoxy, Hydroxy, Cyano, Nitro, Amino, Dimethylamino oder $R^3$ und $R^4$ zusammen Methylendioxy bedeuten. |

5. Benzylselenobenzoesäuren der allgemeinen Formel II

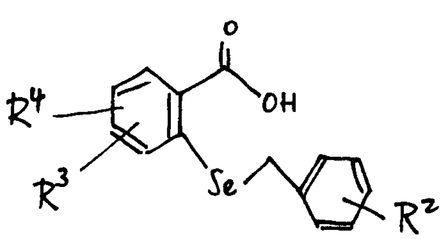

(Formel II)

in der $R^2$, $R^3$ und $R^4$ die gleiche Bedeutung wie in Formel I haben und die als Ausgangsprodukt für die Synthese der Verbindungen der Ansprüche 1 bis 4 verwendet werden.

6. Benzylselenooxazoline der allgemeinen Formel III

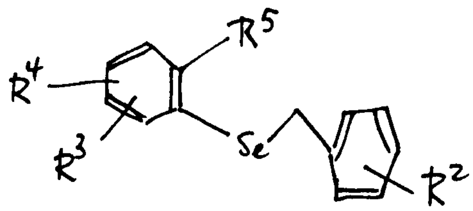

(Formel III)

worin

- $R^2$ Wasserstoff, Fluor, Chlor, Brom, $C_{1-4}$-Alkyl, $C_{1-4}$-Alkoxy oder Dimethylamino und
- $R^3$ unabhängig von $R^4$ Wasserstoff, Fluor, Chlor, Brom, $C_{1-4}$-Alkoxy oder Dimethylamino bedeutet und
- $R^4$ Wasserstoff bedeutet oder zusammen mit $R^3$ für Methylendioxy steht und
- $R^5$ 1,3-Oxazolin-2-yl oder 4,4-Dimethyl-1,3-oxazolin-2-yl bedeutet.

7. Verfahren zur Herstellung von Verbindungen der Formel I gemäß den Ansprüchen 1 bis 4, wobei in der Formel I n = 0 bedeutet, dadurch gekennzeichnet, daß man in einem chlorierten Kohlenwasserstoff eine suspendierte Verbindung der Formel II mit Bis-(2-oxo-3-oxazolidinyl)-phosphorylchlorid und einem Anilin umsetzt und das entstandene Produkt aus dem Reaktionsgemisch abtrennt.

8. Verfahren zur Herstellung von Verbindungen der allgemeinen Formel II gemäß Anspruch 5, dadurch gekennzeichnet, daß man eine Diselenosalicylsäure (Formel IV)

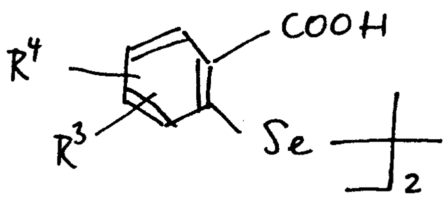

(Formel IV)

wobei $R^3$ und $R^4$ die gleiche Bedeutung haben wie in Formel I in alkalischer Lösung mit einem Benzylbromid umsetzt, mit Salzsäure neutralisiert und das entstandene Produkt aus dem Reaktionsgemisch abtrennt.

9. Verfahren zur Herstellung der Verbindungen der allgemeinen Formel III gemäß Anspruch 6, dadurch gekennzeichnet, daß man die Oxazolin-Verbindung der allgemeinen Formel V

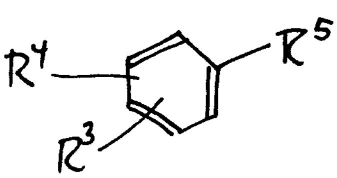

(Formel V)

worin
$R^3$ und $R^4$ die in Formel I angegebene Bedeutung haben und $R^5$ die in Formel III angegebene Bedeutung hat in Tetrahydrofuran - unter Zusatz von n-Butyllithium in Hexan - mit Dibenzyldiselenid umsetzt und das Produkt aus dem Reaktionsgemisch abtrennt.

10. Pharmazeutische Präparate, dadurch gekennzeichnet, daß sie entzündungshemmende Eigenschaften besitzen und als Wirkstoff eine Verbindung der Formel I gemäß den Ansprüchen 1 bis 4 im Gemisch mit üblichen pharmazeutischen Hilfs- und Trägerstoffen enthalten.

## Claims

1. Benzylselenobenzamides of the general formula I

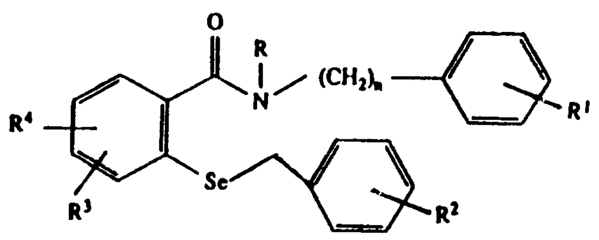

(formula I)

where

| | |
|---|---|
| R | is hydrogen, methyl or ethyl, and |
| $R^1$, $R^2$ | are the same or different and represent independent of each other hydrogen, fluorine, chlorine, bromine, $C_{1-4}$-alkyl, $C_{1-4}$-alkoxy, hydroxy, cyano, amino, dimethylamino or nitro and |
| $R^3$, $R^4$ | are the same or different and, taken separately, represent independent of each other hydrogen, fluorine, chlorine, bromine, $C_{1-4}$-alkyl, $C_{1-4}$-alkoxy, hydroxy, cyano, or nitro and, taken together, represent methylenedioxy and |
| n | is zero, 1 or 2. |

2. Benzylselenobenzamides according to general formula I, claim 1, where

| | |
|---|---|
| n | equals zero and |
| R, $R^2$, $R^3$ and $R^4$ | represent hydrogen, while |
| $R^1$ | is hydrogen, fluorine, chlorine, bromine, methyl, methoxy, hydroxy, cyano or nitro. |

3. Benzylselenobenzamides according to general formula I, claim 1, where

| | |
|---|---|
| n | equals zero and |
| R, $R^1$, $R^3$ and $R^4$ | represent hydrogen, |

while

$R^2$ represents hydrogen, fluorine, chlorine, bromine, methyl, methoxy, hydroxy, cyano or nitro.

4. Benzylselenobenzamides according to general formula I, claim 1, where

| | |
|---|---|
| n | equals zero and |
| R, $R^2$ | represent hydrogen, |

while

| | |
|---|---|
| $R^1$, $R^3$ and $R^4$ | are the same or different and, taken separately, represent independent of each other hydrogen, fluorine, chlorine, bromine, methyl, methoxy, hydroxy, cyano, nitro, amino or dimethylamino and, taken together, represent methylenedioxy. |

**5.** Benzylselenobenzoic acids according to general formula II

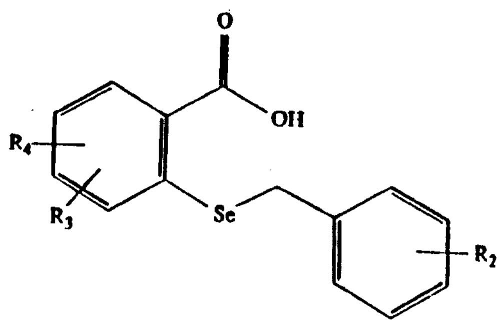

(formula II)

where $R^2$, $R^3$ and $R^4$ possess the meaning assigned in formula I and which are employed as starting materials for the synthesis of compounds according to claims 1 to 4.

**6.** Benzylselenooxazolines of general formula III

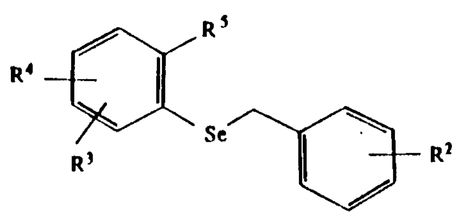

(formula III)

where

- $R^2$ represents hydrogen, fluorine, chlorine, bromine, $C_{1-4}$-alkyl, $C_{1-4}$-alkoxy or dimethylamino and
- $R^3$ independent of $R^4$, represents hydrogen, fluorine, chlorine, bromine, $C_{1-4}$-alkoxy or dimethylamino and
- $R^4$ represents hydrogen or, together with $R^3$, methylenedioxy and
- $R^5$ represents 1,3-oxazolin-2-yl or 4,4-dimethyl-1,3-oxazolin-2-yl.

**7.** Methods of preparation of the compounds of formula I according to claims 1 to 4 whereby in formula I n equals zero, wherein a compound of formula II is suspended in a chlorinated hydrocarbon and reacted with bis-(2-oxo-3-oxazolidinyl) phosphoryl chloride and an aniline and the product so formed is isolated from the reaction mixture.

**8.** Methods of preparation of compounds of general formula II according to claim 5, wherein a diselenosalicylic acid (formula IV)

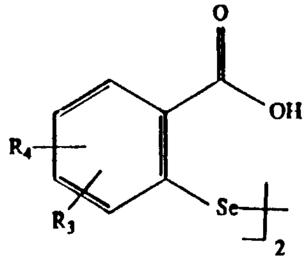

(formula IV)

whereby $R^3$ and $R^4$ have the same meaning as in formula I, is reacted with benzyl bromide in alkaline solution, neutralized with hydrochloric acid and the product that results is isolated from the reaction mixture.

9. Methods of preparation of compounds of general formula III according to claim 6, wherein an oxazoline compound of general formula V,

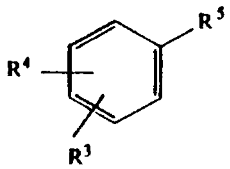

(formula V)

where $R^3$ and $R^4$ have the same meaning as in formula I, and $R^5$ has the meaning assigned in formula III, is reacted in tetrahydrofuran with the dibenzyl diselenide with the addition of n-butyllithium in hexane and the product isolated from the reaction mixture.

10. Pharmaceutical anti-inflammatory products which contain a compound of formula I according to claims 1 to 4 as the active ingredient in admixture with usual pharmaceutical additives and excipients.

**Revendications**

1. Benzylsélénobenzamides de la formule générale I

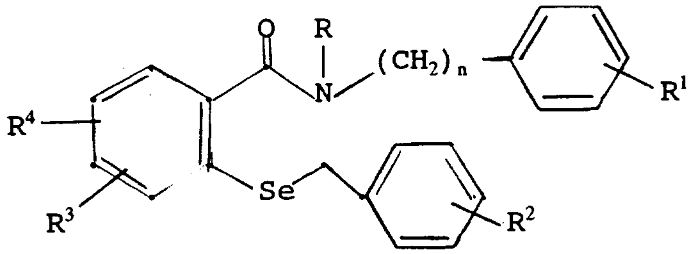

(formule I)

dans laquelle

| | |
|---|---|
| R | représente un atome d'hydrogène, le radical méthyle ou éthyle et |
| $R^1$ et $R^2$ | ont des significations identiques ou différentes et représentent, chacun indépendamment l'un de l'autre, un atome d'hydrogène, de fluor, de chlore, de brome, un radical alkyle en $C_1$-$C_4$, alcoxy en $C_1$-$C_4$, hydroxyle, cyano, amino, diméthylamino ou nitro et |
| $R^4$ et $R^4$ | ont des significations identiques ou différentes et représentent, chacun indépendamment l'un de l'autre, un atome d'hydrogène, de fluor, de chlore de brome, un radical alkyle en $C_1$-$C_4$, alcoxy en $C_1$-$C_4$, hydroxyle, cyano, nitro, ou forment ensemble le radical méthylène dioxy et |
| n | est égal à 0, 1 ou 2. |

2. Benzylsélénobenzamides de la formule I, suivant la revendication 1, caractérisés en ce que

| | |
|---|---|
| n | est égal à zéro et |
| R, $R^2$, $R^3$ et $R^4$ | représentent chacun un atome d'hydrogène, cependant que |
| $R^1$ | représente un atome d'hydrogène, de fluor, de chlore, de brome, le radical méthyle, méthoxy, hydroxyle, cyano ou nitro. |

3. Benzylsélénobenzamides de la formule générale I, suivant la revendication 1, caractérisé en ce que

| | |
|---|---|
| n | est égal à zéro et |
| R $R^1$, $R^3$ et $R^4$ | représentent chacun un atome d'hydrogène, cependant que |
| $R^2$ | représente un atome d'hydrogène, de fluor, de chlore, de brome, le radical méthyle, méthoxy, hydroxyle, cyano ou nitro. |

4. Benzylsélénobenzamides de la formule générale I, suivant la revendication 1, caractérisés en ce que

| | |
|---|---|
| n | est égal à zéro et |
| R $R^2$ | représentent chacun un atome d'hydrogène, cependant que |

$R^1$, $R^3$ et $R^4$ possèdent des significations identiques ou différentes et représentent, chacun indépendamment l'un de l'autre, un atome d'hydrogène, un atome de fluor, de chlore, de brome, le radical méthyle, méthoxy, hydroxyle, cyano, nitro, amino, diméthylamino, ou bien

$R^3$ et $R^4$ représentent ensemble le radical méthylènedioxy.

**5.** Acides benzylsélénobenzoïques de la formule générale II

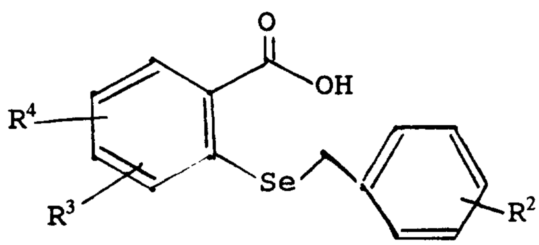

(formule II)

dans laquelle $R^2$, $R^3$ et $R^4$ possèdent les mêmes significations que celles qui leur ont été attribuées à propos de la définition de la formule I et que l'on utilise à titre de produits de départ pour la synthèse des composés suivant l'une quelconque des revendications 1 à 4.

**6.** Benzylsélénooxazolines de la formule générale III

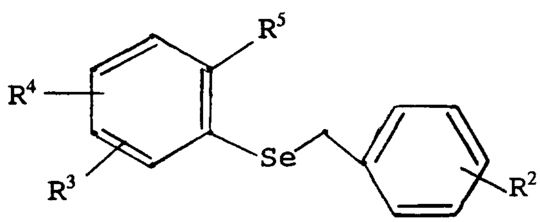

(formule III)

dans laquelle

- $R^2$ représente un atome d'hydrogène, de fluor, de chlore, de brome, un radical alkyle en $C_1$-$C_4$, alcoxy en $C_1$-$C_4$, ou diméthylamino et
- $R^3$ représente, indépendamment de $R^4$, un atome d'hydrogène, de fluor, de chlore, de brome, un radical alcoxy en $C_1$-$C_4$ ou diméthylamino et
- $R^4$ représente un atome d'hydrogène, ou forme avec $R^4$ le radical méthylènedioxy et
- $R^5$ représente un radical 1,3-oxazoline-2-yle ou 4,4-diméthyl-1,3-oxazoline-2-yle.

**7.** Procédé de préparation de composés de la formule I suivant l'une quelconque des revendications 1 à 4, où n est égal à 0 dans la formule I, caractérisé en ce que, dans un hydrocarbure chloré, on fait réagir un composé de la formule II en suspension avec le chlorure de bis-(2-oxo-3-oxazolidnyl)-phosphoryle et une aniline et on sépare le produit formé du mélange réactionnel.

**8.** Procédé de préparation de composés de la formule générale II suivant la revendication 5, caractérisé en ce que l'on fait réagir l'acide disélénosalicylique de la formule IV

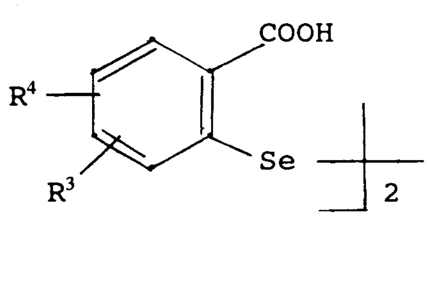

(formule IV)

dans laquelle $R^3$ et $R^4$ possèdent les mêmes significations que celles qui leur ont été attribuées à propos de la définition de la formule I, en solution alcaline, avec le bromure de benzyle, puis on opère une neutralisation à l'acide chlorhydrique et on sépare le produit formé du mélange réactionnel.

**9.** Procédé de préparation des composés de la formule générale III suivant la revendication 6, caractérisé en ce que l'on fait réagir le composé d'oxazoline de la formule V

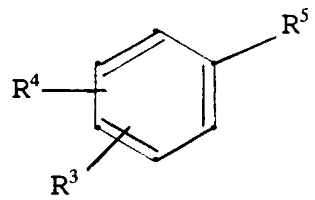

(formule V)

dans laquelle
$R^3$ et $R^4$ possèdent les significations qui leur ont été attribuées à propos de la définition de la formule I et $R^5$ possède les significations qui lui ont été attribuées à propos de la définition de la formule III, dans le tétrahydrofuranne - sous addition de n-butyllithium dans l'hexane - avec le diséléniure de dibenzyle et on sépare le produit du mélange réactionnel.

**10.** Préparations pharmaceutiques, caractérisées en ce qu'elles possèdent des propriétés anti-inflammatoires ou antiphlogistiques et contiennent, à titre de principe actif, un composé de la formule I suivant l'une quelconque des revendications 1 à 4, en mélange à des excipients, véhicules ou adjuvants pharmaceutiques habituels.